# EUROPÄISCHE PATENTSCHRIFT

(11) **EP 0 621 775 B1**
(45) Veröffentlichungstag und Bekanntmachung des Hinweises auf die Patenterteilung: **06.08.1997**
(21) Anmeldenummer: 93902032.7
(22) Anmeldetag: 18.01.1993
(51) Int. Cl.: A61K 9/16, A61K 9/20

(54) **PEPTIDARZNEISTOFFE ENTHALTENDE PELLETS UND IHRE HERSTELLUNG SOWIE DEREN VERWENDUNG**
PELLETS CONTAINING PEPTIDE DRUGS, THEIR MANUFACTURE AND USE
COMPRIMES CONTENANT DES MEDICAMENTS PEPTIDIQUES, LEUR FABRICATION ET LEUR APPLICATION

(30) Priorität: 17.01.1992 DE 4201179; 30.04.1992 US 876865
(43) Veröffentlichungstag der Anmeldung: 02.11.1994
(73) Patentinhaber: ALFATEC-PHARMA GmbH, 69120 Heidelberg (DE)
(72) Erfinder: WUNDERLICH, Jens-Christian, D-6900 Heidelberg (DE); SCHICK, Ursula, D-69198 Schriesheim (DE); WERRY, Jürgen, D-6700 Ludwigshafen (DE); FREIDENREICH, Jürgen, D-6905 Schriesheim (DE)
(74) Vertreter: KUHNEN, WACKER & PARTNER
(86) Internationale Anmeldenummer: DE9300036
(87) Internationale Veröffentlichungsnummer: WO9313753

(56) Entgegenhaltungen:
- EP-A- 0 138 216
- EP-A- 0 230 647
- WO-A-85/05029
- WO-A-89/03207
- WO-A-91/06282
- WO-A-91/09591
- FR-A- 1 259 081
- GB-A- 2 176 105
- US-A- 3 312 594

## Beschreibung

Die Erfindung betrifft eine perorale Applikationsform für Peptidarzneistoffe in Form von Pellets, die mindestens einen Peptidarzneistoff in einer Matrix aus Kollagen oder Gelatine, sowie Weichmacher, verteilt enthalten, wobei die Matrix eine halbfeste bis gelförmige Konsistenz aufweist. Die Erfindung betrifft weiterhin ein Verfahren zur Herstellung solcher Pellets, die Peptidarzneistoff(e) enthalten.

Eine optimale, für den Patienten einfache und sichere Anwendung von Arzneistoffen stellt heute die perorale Applikation, beispielsweise von Tabletten, Kapseln oder Trinklösungen dar. Im Falle von Peptidarzneistoffen, wie z.B. Insulin, ergeben sich aber ausgeprägte Schwierigkeiten, weil diese Stoffe nach Freisetzung im Gastrointestinaltrakt (Magen oder Dünndarm) bereits vor der Resorption durch enzymatischen Abbau zum größten Teil inaktiviert werden. Enzymatischer Abbau in der Magen- oder Dünndarmflüssigkeit oder auf der Mucosa droht die Bioverfügbarkeit von Peptidarzneistoffen, besonders Insulin, auf ein Minimum zu senken.

Außerdem entfällt für Peptidarzneistoffe der Resorptionsmechanismus durch passiven Transport weitgehend. Substanzspezifische Eigenschaften, wie Hydrophilie (niedriger Verteilungskoeffizient), Selbstassoziation zu größeren Einheiten oder Bindung an Bestandteile des Gastrointestinaltrakts (GIT) erschweren die Resorption. Ferner wird die Resorption zusätzlich erschwert, wenn durch Dissoziation funktioneller Wirkstoffgruppen entstehende negative Ladung zu elektrostatischer Abstoßung an der Glycocalyx führt, der negativ geladenen Glykoproteinschicht, die der Lipiddoppelschicht aufliegt. Resorption von Peptidarzneistoffen ist aber trotz dieser Schwierigkeiten gerade dann von außerordentlicher Bedeutung, wenn man eine parenterale Zufuhr erfolgreich umgehen will.

Dieses Hauptproblem führt dazu, daß bis jetzt Peptidarzneistoffe grundsätzlich auf keinem anderen Weg als auf dem parenteralen therapeutisch sinnvoll zu applizieren sind.

Ähnlich stellt sich die Situation dar, wenn man alternative Applikationswege wie die bukkale, sublinguale, nasale oder rektale Applikation zu beurteilen hat. Das Hauptproblem ist stets die Überwindung der natürlichen Resorptionsbarriere (Schleimhaut bzw. Mucosa). Daher werden auch bei derartigen Applikationswegen die meisten Peptidarzneistoffe entweder gar nicht resorbiert oder bereits vor der Resorption inaktiviert. Dies liegt zum einen ebenfalls an der Molekülgröße bzw. anderen physikalisch, chemischen Eigenschaften wie Löslichkeit, Polarität des Moleküls etc., zum anderen analog an der Inaktivierung durch enzymatischen Abbau im physiologischen Milieu.

Deshalb ist man bei der Suche nach Alternativen zur parenteralen Applikation immer auf ein Vielfaches der Dosis angewiesen, die man bei der Injektion geben würde. Neuentwicklungen für die Applikation im nasalen Bereich sind kritisch zu beurteilen, da bei Langzeitanwendung solcher Nasensprays sich toxische Nebenwirkungen auf die Nasenschleimhaut ergeben können (Entzündungen, Durchblutungsstörungen, Gefahr von Perforationen). Diese Nebenwirkungen werden hauptsächlich durch ungeeignete Penetrations- bzw. Resorptionsbeschleunigersubstanzen, sogenannte Enhancer, hervorgerufen, deren Risikopotential nur schwer abzuschätzen ist. Für die konventionelle nasale Applikation von Peptidarzneistoffen kann jedoch auf den Einsatz dieser Enhancer bis jetzt nicht verzichtet werden, da sonst die Bioverfügbarkeit der Peptidarzneistoffe verschwindend gering wird.

Der vorliegenden Erfindung liegt daher die Aufgabe zugrunde, eine einfache galenische Zubereitung für Peptidarzneistoffe zu entwickeln, die für mehrere Applikationswege darunter, insbesondere den peroralen, gleichermaßen gut geeignet ist, das beschriebene Resorptionsproblem dieser Stoffe hinreichend löst und deren bekannte niedrige Bioverfügbarkeit signifikant zu steigern vermag.

Diese Aufgabe wird erfindungsgemäß durch eine perorale Applikationsform für Peptidarzneistoffe in Form von Pellets gelöst, die gekennzeichnet ist durch eine Dispersion wenigstens eines Peptidarzneistoffs in einer Matrix, die im wesentlichen Gerüstbildner aus hydrophilen Makromolekülen umfaßt, welche ausgewählt sind aus der Gruppe bestehend aus: Kollagen, Gelatine, fraktionierte Gelatine, Gelatinederivaten, Pflanzenproteinen, Pflanzenproteinhydrolysaten, Kollagenderivaten, Kollagenhydrolysaten, Elastinhydrolysaten, sowie deren Mischungen, und welche ferner wenigstens einen Weichmacher umfaßt, wobei die Matrix in fester, halbfester bis gelförmiger Konsistenz vorliegt.

Ferner stellt die vorliegende Erfindung auch ein Verfahren zur Herstellung einer peroralen Applikationsform von Pellets zur Verfügung, die wenigstens einen Peptidarzneistoff enthalten, das dadurch gekennzeichnet ist, daß man
a) den Peptidarzneistoff in einer Matrix aus einem Gerüstbildner aus hydrophilen Makromolekülen, welcher ausgewählt ist aus der Gruppe bestehend aus: Kollagen, Gelatine, fraktionierter Gelatine, Gelatinederivate, Pflanzenproteine, Pflanzen-proteinhydrolysate, Kollagenderivate Kollagen-hydrolysate, Elastinhydrolysate; sowie deren Mischungen; und Weichmacher dispergiert; und
b) die erhaltene Mischung aus Gerüstbildner, Weichmacher und Peptidarzneistoff in ein tiefkaltes inertes verflüssigtes Gas eintropft und damit Pellets formt.

Darüber hinaus stellt die vorliegende Erfindung die Verwendung einer Kombination wenigstens eines hydrophilen Makromoleküls, ausgewählt aus der Gruppe bestehend aus:
Kollagen, Gelatine, fraktionierte Gelatine, Kollagenderivate, Kollagenhydrolysate, Pflanzenproteine, Pflanzenproteinhydrolysate, Elastinhydrolysate; sowie deren Mischungen;
mit einem Weichmacher, der ausgewählt ist aus der Gruppe bestehend aus:
Glycerol, Propylenglykol, Polyethylenglykole, Triacetin, Sorbitol, Sorbitangemische, Sorbitollösungen, Glucosesirup, Polyole, Zuckeralkohole; sowie deren Mischungen, als Enhancer in einer der vorgeschlagenen peroralen Applikationsform zur Verfügung. Als hydrophile Makromoleküle, die als Gerüstbildner für die erfindungsgemäße Matrix eingesetzt werden, sind zu nennen: Kollagenhydrolysate, Gelatinederivate, Pflanzenproteine, Pflanzenproteinhydrolysate, Elastinhydrolysate, Kollagen, Gelatine, fraktionierte Gelatine, Kollagenderivate.

Gelatine ist ein aus kollagenhaltigem Material gewonnenes Skleroprotein, das je nach Herstellungsprozeß unterschiedliche Eigenschaften hat. Sie besteht im wesentlichen aus vier Molekulargewichtsfraktionen, die die physikalisch-chemischen Eigenschaften in Abhängigkeit vom Molekulargewicht und prozentualem Gewichtsanteil beeinflussen. Je höher z.B. der Anteil Mikrogel (10⁷ bis 10⁸ Dalton) liegt, desto höher ist auch die Viskosität der wäßrigen Lösung. Handelsübliche Sorten enthalten bis zu 15 Gewichtsprozent. Die Fraktionen der alpha-Gelatine und deren Oligomere (9,5 x 10⁴ / 10⁵ bis 10⁶ D) sind entscheidend für die Gelfestigkeit und liegen üblicherweise zwischen 10 und 40 Gewichtsprozent. Molekulargewichte unterhalb der alpha-Gelatine werden als Peptide bezeichnet und können in herkömmlichen Gelatinequalitäten (niedrigbloomig) bis zu 80 Gewichtsprozent betragen.

Gelatine besitzt ein temperatur- und konzentrationsabhängiges Sol-Gel-Umwandlungsverhalten, das von der molekularen Zusammensetzung abhängig ist. Als Konventionsmethode für das Gelbildungsvermögen wird die Bloomzahl angegeben. Niedrige Handelsqualitäten beginnen bei 50 Bloom, hochbloomige Sorten liegen bei etwa 300 Bloom.

Fraktionierte Gelatine stellt den Spezialfall von Gelatine dar und wird durch spezielle Herstellungstechniken, wie z.B. Ultrafiltration aus herkömmlicher Gelatine gewonnen. Die Zusammensetzung kann z.B. durch Entfernung von Peptiden (MG < 9,5 x 10⁴D) oder durch Mischungen aus Einzelfraktionen wie z.B. alpha-Ketten, dimeren und trimeren Ketten oder Mikrogel variiert werden.

Kollagen in nativer Form ist wasserunlöslich. Durch spezielle Herstellungsverfahren gibt es heute lösliche Kollagentypen.

Kollagenderivate sind veränderte Kollagenmoleküle, die beispielsweise mit Crosslinkern dreidimensional vernetzt sein können oder z.B auf andere Art und Weise chemisch vernetzt sein können.

Unter Kollagenhydrolysat wird ein von Kollagen oder Gelatine druckhydrolytisch oder enzymatisch gewonnenes Produkt verstanden, das leicht kaltwasserlöslich ist und eine Molekulargewichtszusammensetzung zwischen einigen hundert D bis 9,5 x 10⁴ D aufweist.

Elastinhydrolysate werden enzymatisch aus Elastin gewonnen und besitzen einen hohen Anteil an nicht polaren Aminosäuren. Sie weisen ein Molekulargewicht von ca. 2.000 bis 3.000 D auf.

Pflanzenproteine bzw. deren Hydrolysate werden vorzugsweise aus Weizen und Soja gewonnen und besitzen beispielsweise Molekulargewichte von 200.000 bis 300.000 D bzw. 1.000 bis 10.000 D.

Die Zusätze von Weichmachern liegen bei 1-50% (bezogen auf die zu verarbeitende Masse) und werden ausgewählt aus der Gruppe bestehend aus: Glycerol, Propylenglykol, Polyethylenglykole, Triacetin, Sorbitol, Sorbitangemische, Sorbitollösungen, Glucosesirup und andere Polyole bzw. Zuckeralkohole; und deren Mischungen.

Gemäß der vorliegenden Erfindung werden unter Pellets bzw. Formkörpern sowohl Formkörper mit einer Größe bis zu ca. 2 mm verstanden, als auch mit einer Größe bis zu 12 mm. Letztere "Pellets" sind als einzeldosierbare Vollkugeln anzusehen.

Weitere Ausführungen hierzu sind in den im folgenden aufgelisteten parallelen internationalen (PCT)-Anmeldungen enthalten. Die Inhalte dieser parallelen PCT-Anmeldungen, am selben Tage beim Deutschen Patentamt von denselben Erfindern und Anmeldern eingereicht:
PCT/DE93/00037, entsprechend WO 93/13754
PCT/DE93/00038, entsprechend WO 93/13757
PCT/DE93/00035, entsprechend WO 93/13761
werden hiermit ebenso vollinhaltlich zur Offenbarung der vorliegenden Anmeldung gemacht, wie die älteren PCT-Anmeldungen:
PCT/DE92/01010, PCT/DE92/01012, PCT/DE92/01014, PCT/DE92/01016, PCT/DE92/01007, PCT/DE92/01008, PCT/DE92/01015, PCT/DE92/01013, PCT/DE92/01009, PCT/DE92/01011 vom 4.12.1992.

Es hat sich erstaunlicherweise gezeigt, daß die Inkorporierung von Peptidarzneistoffen in eine erfindungsgemäße Pelletmatrix mit halbfester bzw. gelförmiger Konsistenz Vorteile in vielfacher Hinsicht bietet.

Der Peptidarzneistoff liegt in der Matrix geschützt vor äußeren Einflüssen wie z.B. Licht, Luftsauerstoff etc. vor und ist damit in seiner galenischen Zubereitung vor Aktivitätsverlusten durch Zersetzungsprozesse bewahrt.

Ebenso haben eigene Untersuchungen gezeigt, daß für Peptidarzneistoffe, die in den erfindungsgemäßen Pellets inkorporiert vorliegen, eine gesteigerte Resorption und damit eine signifikante Erhöhung der Bioverfügbarkeit bei der Applikation in-vivo zu erwarten ist.

Bei Peptidarzneistoffen, die unter herkömmlichen Bedingungen als schlecht resorbierbar gelten, konnte somit durch Einarbeitung, sogar als einfache Dispersion, in eine erfindungsgemäße Zubereitung eine Bioverfügbarkeitssteigerung von 100 bis 150% erzielt werden, im Vergleich zu einer konventionellen Zubereitung gleicher Dosierung des Peptidarzneistoffs.

Offensichtlich führt also das Vorliegen in einer erfindungsgemäßen Zubereitung zu einer stark erhöhten (effektiveren) Resorption der Peptidarzneistoffdosis unter physiologischen Bedingungen.

Als Applikationsarten eignet sich erfindungsgemäß die perorale Applikation.

Um die physiologischen Hintergründe der Resorption von Arzneistoffen im allgemeinen und die verbesserte Resorptionsquote der erfindungsgemäßen Pelletformulierungen ausreichend zu erläutern, ist zunächst eine Betrachtung zum Mechanismus der physiologischen Resorption von Arzneistoffen erforderlich, wie er auch in einschlägigen Publikationen dargestellt wird. Allerdings ist die vorliegende Erfindung weder an den folgenden Versuch einer wissenschaftlichen Erklärung der erfindungsgemäß auftretenden Phenomene gebunden noch kann sie hierdurch eingeschränkt werden.

Die passive Arzneistoffresorption erfolgt nach heutigem Erkenntnisstand (Theorie nach Brodie et al.), wenn folgende Bedingungen vorliegen:
a) die Gastrointestinalmembran wirkt als Lipidbarriere,
b) der Arzneistoff wird nur in gelöster und ungeladener, d.h. nichtionisierter Form aufgenommen,
c) saure Arzneistoffe werden bevorzugt im Magen, basisische Arzneistoffe bevorzugt im Darm resorbiert.

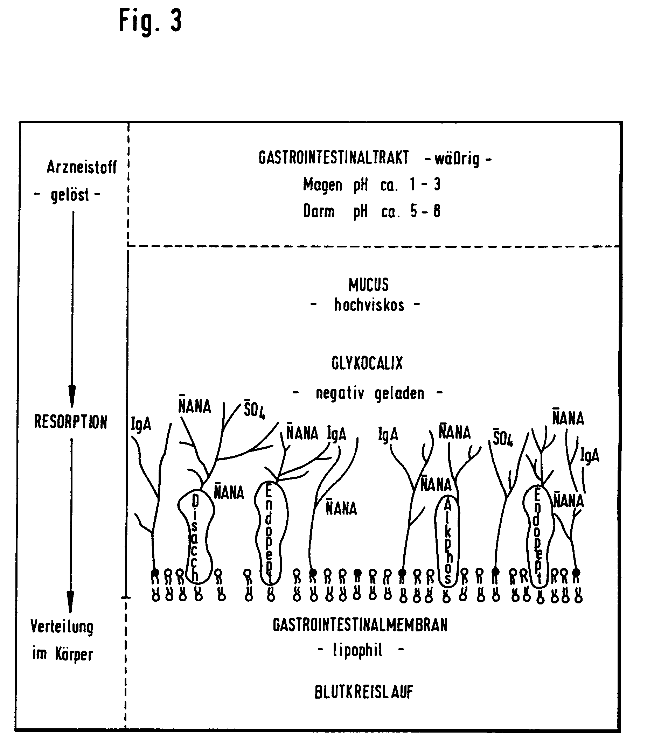

Nach der peroralen Aufnahme eines Arzneistoffs in den Organismus wird seine Resorption, d.h. der Übertritt in den allgemeinen Kreislauf (Biophase) in starkem Maße durch physikalische Barrieren behindert (siehe Fig. 3), nämlich
- durch die Mucus-Schicht und eine wässerige, daran anhärierende Schicht
- die Zellmembranen der intestinalen Epithelzellen mit der daran kovalent gebundenen Glykocalix sowie
- die sogenannten "Tight Junctions", die die Epihelzellen an ihrer apikalen Seite miteinander verbinden.

Diese Barrieren bedingen, daß die Resorption von Arzneistoffen - hauptsächlich abhängig von ihrem Verteilungsmechanismus und Ladungszustand - durch die Lipid-Doppelschichten erfolgt (sogenannte passive Diffusion).

Die Epithelzellen des gesamten Magen-Darm-Traktes sind mit einer Mucus-Schicht bedeckt, die aus Mucinen (Glykoproteinen), Elektrolyten, Proteinen und Nucleinsäuren besteht. Vor allem die Glykoproteine bilden mit dem Hauptanteil des Muscus, nämlich Wasser, eine viskose Gelstruktur, die in erster Linie Schutzfunktionen für die darunter liegende Epithelschicht ausübt. Die Mucusschicht ist an die apikale Oberfläche der Epithelzellen über die Glykocalix gebunden. Die Glykocalix hat ebenfalls eine Glykoproteinstruktur, die kovalent an Bausteine der Membran-Doppelschicht der Epithelzellen gebunden ist. Die verzweigten Polysaccharide der Glykocalix, die entweder direkt an amphiphile Moleküle der Doppelmembran oder an die Doppelmembran inkorporierte Proteine kovalent gebunden sind, besitzen geladene N-Acetyl-Neuraminsäure- und Sulfat-Reste und sind daher negativ geladen, was zu einer elektrostatischen Bindung oder Abstoßung von geladenen Arzneistoffmolekülen bzw. von elektrostatisch geladenen Partikeln führen kann. Die Epithelzellmembranen bestehen aus Phospholipid-Doppelschichten, in die Proteine über ihre hydrophoben Bereiche verankert sind. Die Phosholipid-Doppelschichten mit ihrem lipophilen Anteil stellen eine weitere Barriere für den Transport der zu resorbierenden Arzneistoffe dar.

Aus dieser Darstellung geht deutlich hervor, daß geladene Arzneistoffmoleküle bzw. elektrostatisch geladene Partikel daher nur eine sehr geringe Chance haben, über den peroralen Applikationsweg resorbiert zu werden.

Die erfindungsgemäßen Formkörper geben erstmalig die technische Lehre, ein System zu bilden, mit dem diese vorgenannten Resorptionshindernisse zu überwinden sind.

Hydrophile Makromoleküle, insbesondere Gelatine, sind amphiphile Substanzen, die je nach pH-Wert unterschiedliche Ladungszustände aufweisen. Erfindungsgemäß kann nun das hydrophile Makromolekül in den erfindungsgemäßen Systemen so ausgewählt werden, bzw. der pH-Wert der Formulierung so abgestimmt werden, daß sich im physiologischen Milieu ein positiver Ladungszustand ergibt. Damit läßt sich zumindest eine Teilneutralisation der negativen Oberflächenladungen der Glycocalix erreichen. Dieses Neutralisationsphänomen kann durch bioadhäsive Eigenschaften des hydrophilen Makromoleküls, insbesondere Gelatine noch verstärkt wirksam werden.

Da gelöste Arzneistoffmoleküle nun die Glykocalix ungehindert passieren können, ohne durch elektrostatische Effekte gebunden bzw. abgestoßen zu werden, erreichen sie damit auch die Oberfläche der Epithelzellen und stehen dort in hoher Konzentration zur Verfügung.

Nun können auch aktive, carriervermittelte Transportmechanismen bzw. Phagozytose einen wesentlichen Beitrag zur Resorption liefern.

Das bereits im Stand der Technik beschriebene Problem, das sich üblicherweise bei diesen angesprochenen Applikationswegen ergibt, ist die resorptionsbegrenzende Eigenschaft der physiologischen Schleimhaut oder der drohende enzymatische Abbau der Peptidarzneistoffe im physiologischen Flüssigkeitsmilieu.

Für die perorale Applikation ergibt sich nun folgendes Bild:

Die zu applizierenden erfindungsgemäßen Pellets sind vorteilhafterweise mit geeigneten Filmüberzügen, z.B. Eudragiten^{R} (Röhm Pharma, Deutschland) magensaftresistent überzogen. Besonders bewährt haben sich Eudragit S, Mischungen aus Eudragit S und RS-Typen, oder Mischungen aus Eudragit S, Eudragit L und Eudragit RS-Typen. Diese Filmüberzüge besitzen den Vorteil, daß sie bis zur Auflösung wasserundurchlässig sind und sich erst bei pH-Werten oberhalb ca. 7 aufzulösen beginnen, also nachdem die Arzneiform sich bereits in unteren Darmabschnitten befindet oder bereits im Colon. Erst ab diesem Zeitpunkt kann dann die Freisetzung des Peptidarzneistoffs durch Schmelzen der Matrixmasse beginnen. Der Peptidarzneistoff ist damit vor dem enzymatischen Abbau durch die Enzyme der Verdauungsflüssigkeit so lange wirksam geschützt, bis ein peptidasearmer Resorptionsort wie das Colon erreicht ist.

Statt Eudragiten^{R} können auch geeignete Filmüberzüge aus Substanzen, die nach Erreichen des Colons durch dort vorhandene Enzyme abgebaut werden, eingesetzt werden. Hierbei handelt es sich z.B. um azo-vernetzte Polymethacrylate; Polyurethan-Zucker-Copolymere, wobei als Zuckerkomponente insbesondere oligomere Galactomannane oder Galactomannanderivate geeignet sind, die dann im aliphatischen Diisocyanaten vernetzt werden; Galactomannanderivate wie Ethyl- oder Acetylgalactomannane; mit Adipinsäure vernetzte Polysaccharide.

Statt einzeln zu applizierender Pellets (Vollkugeln) können auch Formkörper mit kleinerem Durchmesser (kleiner ca. 2 mm) in konventionelle Hartgelatinekapseln abgefüllt werden. Diese Kapseln sind üblicherweise mit den gleichen, oben beschriebenen Filmbildnern überzogen, um mindestens eine Magensaftresistenz zu erreichen, bevor der Kapselinhalt (einzelne Pellets) freigegeben wird.

Nach dem Schmelzen der erfindungsgemäßen Pelletmatrix im physiologischen Milieu und beginnender Arzneistofffreisetzung schützt die umhüllende (viskose) Solschicht den Peptidarzneistoff wirksam vor dem enzymatischen Abbau durch noch vorhandene Peptidasen. Ein Diffundieren der abbauenden Enzyme durch diese Solschicht ist nämlich erschwert.

Desweiteren zeigt die geschmolzene Matrixmasse hohe Affinität zur Schleimhautoberfläche. Dieses Anheften oder Ankleben an der Schleimhaut (Bioadhäsivität) bewirkt einen direkten Kontakt des Peptidarzneistoffs mit der physiologischen Resorptionsbarriere. Überraschenderweise hat sich dabei gezeigt, daß die Gelatine/Weichmacher-Kombination als sogenannter "Enhancer" wirkt. Dadurch wird der Resorptionsprozess erheblich erleichtert bzw. beschleunigt und vor allem signifikant gesteigert gegenüber herkömmlichen Zubereitungen.

Daher stellt die vorliegende Erfindung auch die Verwendung der oben genannten Kombination als Enhancer in der erfindungsgemäßen peroralen Applikationsform zur Verfügung.

Ein Wiederausfallen gelöst in der Matrix vorliegender Peptidarzneistoffe im relativ flüssigkeitsarmen Colon wird durch die guten Puffereigenschaften der Gelatine verhindert. Diese Tatsache kann durch Einarbeiten von Puffersubstanzen (z.B. Dinatriumhydrogenphosphat) in die erfindungsgemäße Pelletmatrix noch verstärkt werden.

Eine besondere Ausführungsform der vorliegenden Erfindung, die in Unteransprüchen beansprucht ist, ergibt sich dann, wenn in der Pelletmatrix zusätzliche Hilfsstoffe (Penetrationsbeschleuniger oder sogenannte Enhancer) vorliegen. Diese Stoffe treten in Wechselwirkung mit den Membranen an Schleimhautoberflächen und beschleunigen die erfindungsgemäße Steigerung der Resorptionsrate von Peptidarzneistoffen noch weiter. Gelatine vermindert dabei wirksam das schleimhautirritierende Potential dieser Enhancersubstanzen. Solche Enhancer sind vielfach Tenside (z.B. Natriumlaurylsulfat), von denen die haut- und schleimhautirritierende Wirkung hinreichend bekannt ist. Tenside bilden Komplexe mit proteinhaltigen Bestandteilen von Haut und Schleimhaut. Durch Komplexbildung zwischen Gelatine als proteinhaltigem Material und ionischem Tensid wird jedoch die Reizwirkung solcher Tenside auf die Schleimhaut vermindert. Diese Komplexbildung ist umso stärker, je polarer beispielsweise ein Tensidanion (z.B. das hier erwähnte Natriumlaurylsulfat ) ist.

Folglich ergibt sich, daß die Bioverfügbarkeit solcher Zubereitungen gegenüber herkömmlichen Zubereitungen damit nochmals erhöht werden kann, bei vermindertem Reizpotential.

Die erfindungsgemäßen Pellets stellen runde, einheitliche Formkörper mit Korngrößen im Bereich von 0,2 - 12 mm dar. Sie weisen eine hohe Festigkeit auf, sind lagerstabil, gut dosierbar und fallen, bedingt durch den besonderen Herstellungsprozeß, als freifließendes Gut an.

Die Konsistenz der erfindungsgemäßen Pellets steht in direkter Abhängigkeit von dem eingesetzten Sol-Gel-Bildner und der Art und Konzentration des Weichmacherzusatzes.

Der Einsatz von höherbloomigen Gelatinesorten in den erfindungsgemäßen Formkörpern (Bloomwert oberhalb ca. 250) führt zu ganz besonderen Eigenschaften hinsichtlich des bioadhäsiven Verhaltens. Solche Formkörper quellen unter physiologischen Bedingungen durch Feuchtigkeitsaufnahme an der Oberfläche zunächst an. Auflösung der Matrixmasse erfolgt nur sehr langsam und verzögert. Diese sich ausbildende äußere Solschicht mit sehr hoher Viskosität zeigt ausgezeichnete Haft- und Klebwirkung an Schleimhautoberflächen.

Erfindungsgemäße Formkörper mit niedrigbloomigeren Gelatinesorten (unterhalb 250 Bloom) zeigen dagegen unter physiologischen Bedingungen eine schnellere Auflösung der Matrixmasse. Nach dem Schmelzen führt aber hier der Glycerolzusatz zur Ausbildung einer zähen Masse mit hoher Viskosität, die als flächenförmiges Gebilde über die Schleimhaut verteilt ist und an dieser haftet bzw. klebt.

Bei Peptidarzneistoffen, die extrem thermolabil sind, ermöglicht die Erfindung in einer weiteren Ausführungsform überraschenderweise Formkörper mit den erfindungsgemäßen Eigenschaften zur Verfügung zu stellen, die unter ausschließlich kalten Bedingungen hergestellt sind. Bei dieser Vorgehensweise verwendet man eine Matrix aus einem hydrophilen Makromolekül ausgewählt aus der Gruppe bestehend aus: Pflanzenproteine, Pflanzenproteinhydrolysate, Elastinhydrolysate, Kollagenhydrolysate, kaltwasserlösliche Gelatine, Gelatinederivate; und Mischungen der vorgenannten Stoffe; mit einem Maximum der Molekulargewichtsverteilung unterhalb 10⁵ D.

Besonders geeignet als Weichmacherzusätze sind Stoffe wie z.B. Sorbitol, die nach der Trocknung bei Raumtemperatur fest sind. Erstaunlicherweise bildet die Matrix solcher Pellets nach der Lyophilisation eine feste bis halbfeste Struktur aus, die nach Kontakt mit wäßrigem Medium bzw. unter physiologischen Bedingungen eine bioadhäsive und hochviskose Eigenschaft im Sinne der Erfindung ergibt. Zu der aus hydrophilen Makromolekülen aufgebauten Matrix können weitere pharmazeutisch akzeptable Hilfs- oder Trägerstoffe in Abstimmung auf den jeweiligen Peptidarzneistoff von ca. 1-50% (Gewichtsprozent, bezogen auf die zu verarbeitende Masse) zugesetzt werden.

Im einfachsten Fall läßt sich das erfindungsgemäße Verfahren zur Herstellung von Peptidarzneistoff(en) enthaltenden Pellets mit den folgenden zwei Verfahrensschritten beschreiben:
a) Man dispergiert einen Peptidarzneistoff in einer Matrix aus einem Gerüstbildner aus hydrophilen Makromolekülen aus der Gruppe: Kollagen, Gelatine, fraktionierte Gelatine, Kollagenderivate, und Weichmacher.
b) Man tropft die erhaltene Mischung aus Gerüstbildner, Weichmacher und Peptidarzneistoff(en) in eine tiefkalte inerte Flüssigkeit ein und formt damit Pellets.

Bei einer Ausführungsform des unter a) beschriebenen Verfahrensschrittes stellt man eine tropffähige Masse, vorwiegend bestehend aus hydrophilen Makromolekülen als Gerüstbildner, insbesondere Gelatine her.

Dabei wird die Gelatine zunächst mit Wasser bei einer Temperatur von 30°C bis 50°C in die Solform überführt. Die Konzentration des Trägermaterials kann beispielsweise von 0,5 bis 60% (Gewichtsprozent, bezogen auf die zu verarbeitende Masse), bevorzugt 0,5 bis 30% variieren.

Der pH-Wert des Systems wird durch Zusatz von Säure (z.B. Salzsäure) bzw. Base auf einen Wert eingestellt, der es ermöglicht, den Peptidarzneistoff, beispielsweise Insulin, direkt in der Matrixmasse unter Rühren zu lösen.

Insulin ist aufgrund seines isoelektrischen Punktes (IEP) von 5,3-5,4 im schwach sauren Milieu bei pH 3-4, sowie im schwach alkalischen Milieu bei pH 7-8 ausreichend löslich und auch hinreichend stabil.

Andere Peptidarzneistoffe mit geringerer Löslichkeit in Wasser, z.B. Ciclosporin können dem Gelatinesol bereits in gelöster Form zugesetzt werden. Die Wasserlöslichkeit von Ciclosporin beträgt bei 25°C nur 0,04 mg/g. Ein solcher Stoff kann daher beispielsweise als Lösung in Polyethylenglykol oder Olivenöl in der Grundmasse homogenisiert bzw. emulgiert werden. Dafür können geeignete maschinelle Einrichtungen wie z.B. Homogenisatoren, Planetenrührwerke etc. verwendet werden. Bei der Emulsionsherstellung kann die Ölphase vorteilhaft eine zu verwendende Enhancersubstanz (Beschreibung siehe unten) bereits enthalten und mit dieser in der Matrixmasse homogenisiert werden. Aufgrund zusätzlicher Tensidwirkung der Enhancersubstanz wird eine Emulsionsbildung erleichtert.

Auch die Einarbeitung von mikroverkapselten Peptidarzneistoffen oder Koazervaten in die Matrixmasse ist möglich.

Desweiteren können auch Mikroemulsionen mit der erfindungsgemäßen Matrixmasse vermischt werden. Unter Mikroemulsionen versteht man beispielsweise Öl in Wasser Emulsionen mit Teilchengrößen im Nanometerbereich. Die wäßrige Phase kann einen löslichen Peptidarzneistoff enthalten, die Ölphase wiederum die Enhancersubstanz(en). Eine mögliche Zusammensetzung der Ölphase umfaßt Cholesterol, Lecithin und nicht veresterte Fettsäuren.

Ganz besonders vorteilhaft ist jedoch erfindungsgemäß der Zusatz von Calciumsalzen (z.B. 1-2% Calciumchlorid) zur Gelatinemasse oder die Verwendung einer Gelatine mit höherem Gehalt an Calciumsalz (nicht völlig von Fremdionen befreite Gelatine). Die Calciumionen bewirken durch Wechselwirkung mit den funktionellen Gruppen der Gelatine einerseits und den polaren Gruppen eines Peptidarzneistoffs andererseits eine Konjugat-(Komplexbildung) der beiden Komponenten. Dadurch können schwer wasserlösliche Peptidarzneistoffe in eine leichter lösliche Form überführt werden. Außerdem fördern die Calciumionen durch Komplexbildung mit Bestandteilen der Zellmembran die Resorption der Peptidarzneistoffe.

Als Arzneistoffe sind erfindungsgemäß einsetzbar:
Reguläres Insulin, mit Zink komplexiertes Insulin oder auch Globin-Zink-Insulin, Octreocid, Desmopressin, Vasopressin, Triptorelin, körpereigene Peptidhormone wie Gonadotropin Releasing Hormon, Somatotropin Releasing Hormon, Corticotropin Releasing Hormon oder Thyreotropin Releasing Hormon, Polypeptidantibiotika, Ciclosporin, Buserelin, Calcitonin, Gonadorelin, Lysoprenin, Oxytocin, Protirelin, Hirudin, Glucagon, Enkephalin oder adrenocorticotropes Hormon, Erythropoietin, Stoffe zur Behandlung von AIDS (Renin-Antagonisten), Stoffe zur Behandlung von Hypertonie (Renin-Antagonisten, Enalapril, Captopril), Interferone (z.B. alpha-Interferon), Antibiotika die sich von Aminosäuren ableiten, Penicilline (z.B. Ampicillin), Cephalosporine (z.B. Cefalexin), Carbapeneme (Thienamycin), Impfstoffe.

Als Enhancer können Stoffe aus der Gruppe:
Polyethylenglykol, Propylenglykol, Mono- und Diglyceride, Polyalkohole, Lecithin, Bacitracin, Polyoxyethylen-9-laurylether, Natriumlaurylsulfat, Natriumtaurocholat, Natriumglykocholat, Natriumcholat, Natriumtaurodesoxycholat, Natriumglykodesoxycholat, Natriumdesoxycholat, Natriumtaurodihydrofusidat, Ethylendiamintetraessigsäure-Dinatrium, Citronensäure, Dimethyl-beta-Cyclodextrin, Ölsäure, Protease-Inhibitoren, Polyacrylsäure verwendet werden.

Die Konzentrationsbereiche dieser Enhancer liegen zwischen 0,1 bis 25% (Gewichtsprozent, bezogen auf die zu verarbeitende Masse), insbesondere zwischen 0,1 bis 10%.

Zu der erfindungsgemäßen Grundmasse können weitere, für die pharmazeutische Anwendung geeignete Hilfs- und Trägerstoffe, wie z.B. zusätzliche Gerüstbildner, die unten stehend näher beschrieben werden, Füllstoffe, wie z.B. Lactose, Dispergiermittel, wie z.B. Dinatriumphosphat, pH-Korrigentien, wie z.B. Dinatriumcitrat, Stabilisatoren, wie z.B. Ascorbinsäure, Kosolventien, wie z.B. Alkohole, natürliche Farbstoffe, wie z.B. Carotinoide, aromatisierende Stoffe oder Geschmackskorrigentien, wie z.B. künstliche Süßstoffe, zugesetzt werden.

Als zusätzliche Gerüstbildner können eingesetzt werden:
Elastin, Elastinhydrolysate, Kollagenhydrolysate, Pflanzenproteine, Pflanzenproteinhydrolysate, Albumin, Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Dextran, Zucker, Glycin, Lactose, Mannit, Polyvinylpyrrolidon, Polyacrylsäure, Polymere aus Methacrylsäure, Polymere aus Methacrylsäureestern; azo-vernetzte Polymethacrylate; Polyurethan-Zucker-Copolymere, wobei als Zuckerkomponente insbesondere oligomere Galactomannane oder Galactomannanderivate geeignet sind, die dann mit aliphatischen Diisocyanaten vernetzt werden; Galactomannanderivate wie Ethyl- oder Acetylgalactomannane; mit Adipinsäure vernetzte Polysaccharide; und deren Mischungen.

Unter Kollagenhydrolysat wird ein von Kollagen oder Gelatine druckhydrolytisch oder enzymatisch gewonnenes Produkt ohne Gelbildungsvermogen verstanden. Die Molekulargewichtszusammensetzung kann herstellungsbedingt zwischen einigen Hundert D bis unterhalb von 9,5 x 10⁴D liegen. Kollagenhydrolysate sind kaltwasserlöslich.

Der in Kollagenhydrolysaten vorliegende hohe Anteil an ausgeprägt polaren Aminosäuren (z.B 12% Hydroxyprolin) verstärkt sowohl die Komplexbildung mit etwaigen zugesetzten Enhancersubstanzen, als auch mit dem Peptidarzneistoff selbst.

Weiterhin kann es erwünscht sein, den beschriebenen Matrixmassen lipophile Bestandteile, wie z.B. Phospholipide zur Ausbildung von Liposomen zuzusetzen.

Durch Zusätze von Weichmachern (Glycerol bzw. Sorbit) von 1-50%, bezogen auf die zu verarbeitende Masse, kann der Restfeuchtegehalt der getrockneten Pellets und damit auch deren Konsistenz von fest bis halbfest eingestellt werden.

Selbstverständlich eignen sich die erfindungsgemäßen Mischungen zu einer sofortigen Abfüllung in flüssiger Form nach dem unter a) beschriebenen Verfahrensschritt zur Ausformung in Behältnissen, wie z.B. Formen, Weichgelatinekapseln sowie geeignete andere Umhüllungen.

In einer Ausführungsform des unter b) beschriebenen Verfahrensschrittes wird die beschriebene Matrixmasse zur Ausrundung (Formgebung) und Schockfrostung in ein Tauchbad im Bereich von ca. -70°C bis -220°C eingetropft. Als tiefkalte und inerte Flüssigkeit wird vorzugsweise flüssiger Stickstoff eingesetzt, der die Bestandteile der Pellets nicht verändert. In der tiefkalten Flüssigkeit bilden sich runde Formkörper (Pellets), die nach der Trocknung eine mechanisch stabile Matrix ausbilden. Die Formgebung erfolgt über ein geeignetes Dosiersystem. Jeder diskrete Tropfen nimmt dabei einerseits bereits während des freien Falls, andererseits im Tauchbad durch die um ihn entstehende Gashülle bzw. die Grenzflächenspannung System/Gas Kugelgestalt an, bevor ein vollständiges Ausfrieren erfolgt. Gerade dieses schnelle, aber dennoch kontrolliert steuerbare Gefrieren fixiert den gegebenen Zustand des Systems augenblicklich, d.h. es können keine Arzneistoffe in das umgebende Medium diffundieren, gelöste Bestandteile können nicht mehr auskristallisieren, Suspensionen können nicht mehr sedimentieren, thermisch empfindliche oder feuchtigkeitsempfindliche Bestandteile werden cryokonserviert, das Trägergerüst kann nicht zusammenschrumpfen usw. Das Herstellungsverfahren mit einem inerten Flüssiggas hat also keine nachteilige Beeinflussung oder Veränderung des Produkts zur Folge. Von besonderem Vorteil ist somit der Erhalt der gewünschten Eigenschaften. Weiterhin arbeitet das Verfahren lösungsmittelfrei, belastet die Umwelt nicht und kann unter Sterilbedingungen durchgeführt werden.

Als Dosiersysteme eignen sich alle Vorrichtungen, die diskrete, gleichmäßige Tropfen vorherbestimmbarer Größe erzeugen können, z.B. pipettenartige Tropfvorrichtungen, geeignete Sprüh- oder Zerstäubungsdüsen mit Dosierpumpen.

Weiterhin können für das erfindungsgemäße Verfahren Dosiervorrichtungen mit Einstoffdüsen, die das zu tropfende Gut getaktet oder intermittierend ausstoßen, verwendet werden.

Eine weiterhin bevorzugte Ausführungsform des erfindungsgemäßen Verfahrens setzt das von Messer Griesheim GmbH entwickelte Cryopel^{R}-Verfahren (basierend auf DE-OS 37 11 169) ein. In Verbindung mit einer Tauchfrostanlage, der Cryopel^{R}-Anlage, ist die apparative Umsetzung des erfindungsgemäßen Verfahrens in den industriellen Maßstab besonders einfach. Diese Anlage, die mit flüssigem Stickstoff betrieben werden kann, zeichnet sich besonders durch ihre Wirtschaftlichkeit aus. Diese Anlage ist auch für Sterilherstellung geeignet. Kontinuierliche Arbeitsweise bei geringem Wartungs- und Reinigungsaufwand ermöglicht die wirtschaftliche Umsetzung des erfindungsgemäßen Verfahrens in den industriellen Maßstab.

Es zeigt:
Fig. 1 eine schematische Darstellung in Schnittansicht einer Vorrichtung zur Durchführung des erfindungsgemäßen Verfahrens; und
Fig. 2 eine weitere Vorrichtung zur Ausführung des erfindungsgemäßen Verfahrens in schematischer Darstellung.
Fig. 3 schematisch die Vorgänge, die bei der passiven Resorption von Arzneistoffen in der Gastrointestinalmembran ablaufen.

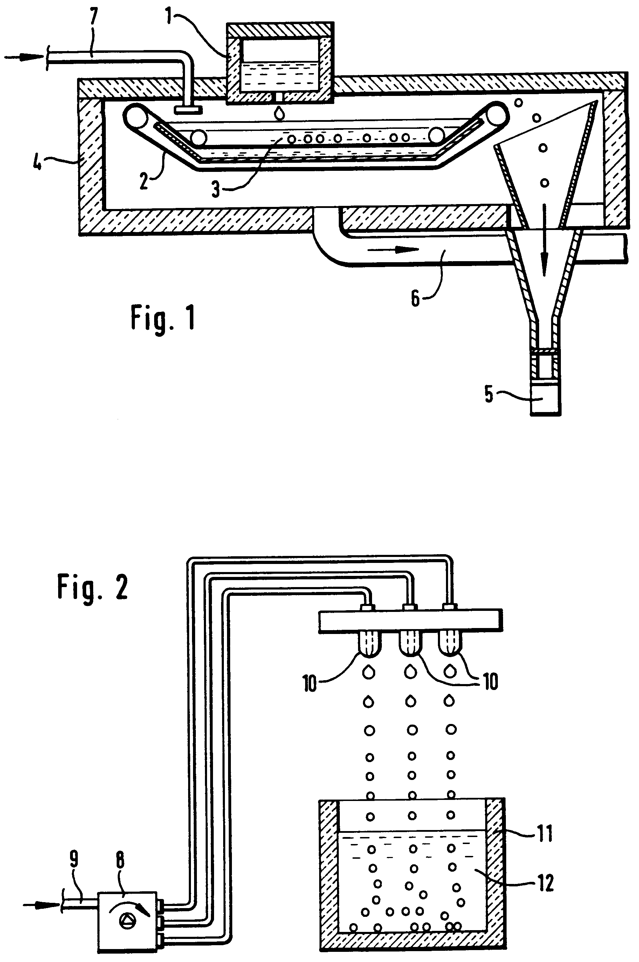

In Fig. 1 ist das von Messer Griesheim GmbH entwickelte Cryopel^{R}-Vefahren schematisch dargestellt. Die erfindungsgemäße Matrixmasse wird aus der beheizbaren Eintragsvorrichtung 1 über kalibrierte Düsen in das Flüssigstickstoffbad 3 bei ca. -196°C eingetropft und unter gleichzeitiger Schockfrostung zu runden Pellets geformt. Über das kontinuierlich laufende Transportband 2 wird das gefrorene Produkt über die Vorrichtung 5 ausgetragen. Die Dosierung des Flüssigstickstoffes erfolgt über die Zuleitung 7 und das entstehende Stickstoffgas entweicht über die Leitung 6. Die Isolierung 4 umschließt das gesamte System.

In Fig. 2 ist eine schematische Darstellung eines Verfahrens gezeigt, bei der über eine regelbare Dosierpumpe 8 die kalte bzw. auf max. 50°C erwärmte Matrixmasse über die Zuleitung 9 kontinuierlich über die beheizbaren Tropfdüsen 10 in die Isolierwanne 11 mit Flüssigstickstoff 12 eintropft. Die schockgefrosteten Pellets werden chargenweise entnommen. Mit dieser Vorrichtung lassen sich hochviskose Massen verarbeiten.

Sollte das zu verarbeitende System nicht ausreichend fließ- bzw. tropffähig sein, kann z.B. weiterer Wasserzusatz von 1-10 Gew.% erfolgen, die Verarbeitungstemperatur erhöht werden oder aber auch Druck bei der Dosierung zur Anwendung kommen. Im umgekehrten Falle (System zu niedrigviskos) ist analog Unterdruck anzuwenden. Auf diese Weise gewährleistet man gleichmäßige Bildung, wie auch Abriß der einzelnen Tropfen.

Die Verarbeitungstemperatur kann in weiten Bereichen variiert werden, soll aber im Falle von thermisch empfindlichen Peptidarzneistoffen unterhalb von 50°C liegen, wobei der Wirkstoff erst unmittelbar vor der Cryopelletierung zugesetzt wird. um deren thermische Belastung zu vermeiden.

Somit können beispielsweise mit einer Cryopel^{R}-Dosiervorrichtung Massen, deren Viskosität sich in einem weiten Bereich bewegte z.B. 1x10⁻³ bis 12,5 Pa x s (Pascalsekunden), problemlos dosiert werden.

Weitere tiefkalte Flüssigkeiten, die sich für das erfindungsgemäße Verfahren eignen, können z.B. flüssige Edelgase wie Argon sein.

In Abhängigkeit vom gewählten Dosiersystem kann eine Korngrößeneinheitlichkeit von über 70% erreicht werden, die sich durch Klassieren noch zusätzlich erhöhen läßt. Durch Klassieren abgetrennte Anteile können erneut in den flüssigen Zustand überführt und wieder pelletiert werden, so daß ein verlustfreies Arbeiten gewährleistet ist.

In einer Ausführungsform des beschriebenen Verfahrensschrittes werden die Pellets getrocknet.

Die gefrorenen Pellets werden aufgetaut und konventionell getrocknet. Hierbei kann vorteilhaft zur Beschleunigung des Trocknungsvorgangs und zur Einhaltung von niedrigen Temperaturen unter Vakuum (3.000-5.000 Pa (30-50 mbar)) gearbeitet werden. Es können Trocknungstemperaturen von bis zu 50°C gewählt werden, wobei die Temperatur während des Trocknungsvorganges in der Pelletmatrix aufgrund der Verdampfungsenthalpie der Flüssigkeit nicht über 30°C ansteigt.

Weiterhin können die in der Verfahrensstufe b) gewonnenen Pellets bzw. Formkörper lyophilisiert werden. Durch das erfindungsgemäße Vorgehen wird das bei ca. -196°C (Schockfrostung) amorph gewordene Wasser bei Temperaturen von ca. 15°C unterhalb des Sublimationspunktes bei einem Druck von ca. 0,1 Pa bis 10³ Pa (ca. 0,001 bis ca. 1,03 mbar) aus der Matrixmasse sublimiert, und es bildet sich ein hochporöses, Mikroporen-haltiges Netzwerk. Es können auch erfindungsgemäß Pellets, die einen Weichmacherzusatz enthalten, gefriergetrocknet werden.

Im Rahmen seines Fachwissens kann der Fachmann leicht selbständig weitere Verfahrensvarianten ausarbeiten. Die nachfolgenden Beispiele sollen die Erfindung näher erläutern:

### Beispiel 1: (perorale Applikation)

- 200 g: Gelatinepulver (220 Bloom)
- 150 g: Glycerol (85%)
- 650 g: destilliertes Wasser

Das Gelatinepulver wird in dem Wasser ca. 45 min vorgequollen und bei 40°C vollständig aufgelöst. Durch Salzsäurezusatz wird ein pH-Wert von 3 eingestellt.

10000 I.E. (internationale Einheiten) Normal-Insulin werden unter Rühren in dem Gelatinesol dispergiert. Danach wird das Glycerol homogen untergemischt.

Über die in Fig.1 dargestellte Cryopel^{R}-Apparatur wird die Mischung bei 40°C in ein Tauchbad mit flüssigem Stickstoff getropft und damit Pellets geformt. Die tiefgefrorenen Formkörper werden anschließend aufgetaut und bei einer Temperatur von 25°C so lange aufgetrocknet, bis eine Pelletmenge von 500 mg (bezogen auf die getrockneten Pellets) genau 10 I.E. Insulin enthält.

Die so getrockneten Pellets werden in konventionelle, opak eingefärbte Hartgelatinekapseln abgefüllt mit einem Füllgewicht von je 500 mg. Anschließend werden die Hartgelatinekapseln durch Aufsprühen einer 6%igen acetonischen Lösung von Eudragit S und Eudragit RS im Verhältnis 3:2 magensaftresistent überzogen.

Unter Testbedingungen (Dissolutiontestapparatur nach USP XXII, Drehkörbchen, 1000 ml 0,1 N Salzsäure, 37°C, 50 rpm) erfolgt während der Testdauer von 120 min kein Kapselzerfall. Nach Austausch des Prüfmediums gegen Phosphatpuffer pH 8 erfolgt jedoch ein Kapselzerfall innerhalb 15 min. Die einzelnen Pellets werden nun freigegeben und schmelzen innerhalb von 30 min vollständig.

### Beispiel 2 (perorale Applikation)

- 350 g: Gelatinepulver (300 Bloom)
- 150 g: Glycerol (85%)
- 50 g: Polyethylenglykol 400
- 1450 g: destilliertes Wasser

Die Gelatine wird in dem Wasser ca. 45 min vorgequollen und anschließend bei 50°C vollständig gelöst.

100 g Ciclosporin werden in der Mischung aus dem Glycerol und dem Polyethylenglykol dispergiert und danach mit der Gelatinelösung homogen vermischt.

Anschließend wird über die in Fig. 2 dargestellte Anlage das auf 50°C erwärmte Gemisch über die Pumpe in das Tauchbad mit flüssigem Stickstoff dosiert und damit Pellets geformt. Die tiefgefrorenen Formkörper werden nach Auftauen bei ansteigender Temperatur zwischen 25°C bis 40°C getrocknet und weisen einen Durchmesser von 6 mm auf.

Die Formkörper werden anschließend durch Aufsprühen einer 5%igen methanolischen Lösung von Eudragit® L magensaftresistent überzogen.

Als einzeldosierte Vollkugeln gegeben, beginnt sich der Filmüberzug unter physiologischen Bedingungen bei pH-Werten oberhalb von 6 aufzulösen. Nach Aufquellen an der Oberfläche und Ausbildung einer Solschicht haften bzw. kleben die Formkörper an der Darmschleimhaut.

## Patentansprüche

1. Verfahren zur Herstellung einer peroralen Applikationsform in Form von Pellets, die wenigstens einen Peptidarzneistoff enthalten, dadurch gekennzeichnet, daß man
a) den Peptidarzneistoff in einer Matrix aus einem Gerüstbildner aus hydrophilen Makromolekülen, welcher ausgewählt ist aus der Gruppe bestehend aus:
Kollagen, Gelatine, fraktionierte Gelatine, Gelatinederivate, Pflanzenproteine, Pflanzen-proteinhydrolysate, Kollagenderivate Kollagen-hydrolysate, Elastinhydrolysate, sowie deren Mischungen, und Weichmacher dispergiert, und
b) die erhaltene Mischung aus Gerüstbildner, Weichmacher und Peptidarzneistoff in ein tiefkaltes inertes verflüssigtes Gas eintropft und damit Pellets formt.

2. Verfahren nach Anspruch 1, dadurch gekennzeichnet, daß man die Dispersion in flüssigen Stickstoff eintropft.

3. Verfahren nach Anspruch 1 oder 2, dadurch gekennzeichnet, daß man aus der Dispersion Tropfen in gleichmäßiger vorherbestimmter Form mittels eines Dosiersystems herstellt.

4. Verfahren nach einen der Ansprüche 1 - 3, dadurch gekennzeichnet, daß der Dispersion aus Gerüstbildner und Peptidarzneistoff Weichmacher, ausgewählt aus der Gruppe bestehend aus: Glycerol, Propylenglykol, Polyethylenglykole, Triacetin, Sorbitol, Sorbitangemische, Sorbitollösungen, Glucosesirup und andere Polyole bzw. Zuckeralkohole, sowie deren Mischungen
in einer Konzentration von 1-50 Gew.-% (bezogen auf die zu verarbeitende Masse) zugesetzt werden.

5. Verfahren nach einen der Ansprüche 1 - 4, dadurch gekennzeichnet, daß man die Dispersion aus Gerüstbildner, Weichmacher und Peptidarzneistoff mit einem Resorptionsbeschleuniger, ausgewählt aus der Gruppe bestehend aus: Polyethylenglykol, Propylenglykol, Mono- und Diglyceride, Polyalkohole, Lecithin, Bacitracin, polyoxyethylen-9-laurylether, Natriumlaurylsulfat, Natriumtaurocholat, Natriumglykocholat, Natriumcholat, Natriumtaurodesoxycholat, Natriumglykodesoxycholat, Natriumdesoxycholat, Natriumtaurodihydrofusidat, Ethylendiamintetraessigsäure-Dinatrium, Citronensäure, Dimethyl-beta-Cyclodextrin, Ölsäure, Protease-Inhibitoren, Polyacrylsäure; sowie deren Mischungen, versetzt.

6. Verfahren nach einen der Ansprüche 1 - 5, dadurch gekennzeichnet, daß man die Matrix mit einem zusätzlichen Hilfs- oder Trägerstoff, ausgewählt aus der Gruppe bestehend aus: Kollagenhydrolysate, Elastin, Elastinhydrolysate, Pflanzenproteine, Pflanzenproteinhydrolysate; sowie deren Mischungen versetzt.

7. Verfahren nach Anspruch 1, dadurch gekennzeichnet, daß man die Pellets nach Stufe b) trocknet.

8. Verfahren nach Anspruch 1, dadurch gekennzeichnet, daß man die Pellets nach Stufe b) gefriertrocknet.

9. Verfahren nach Anspruch 1, dadurch gekennzeichnet, daß als Gerüstbildner Gelatine mit einem Maximum der Molekulargewichtsverteilung oberhalb 10⁵ D bei maximal 40°C eingesetzt wird.

10. Perorale Applikationsform für Peptidarzneistoffe in Form von Pellets, enthaltend eine Dispersion wenigstens eines Peptidarzneistoffs in einer Matrix, die im wesentlichen Gerüstbildner aus hydrophilen Makromolekülen umfaßt, welche ausgewählt sind aus der Gruppe bestehend aus: Kollagen, Gelatine, fraktionierte Gelatine, Gelatinederivate, Pflanzenproteine, Pflanzenproteinhydrolysate, Kollagenderivate Kollagenhydrolysate, Elastinhydrolysate, sowie deren Mischungen, und welche ferner wenigstens einen Weichmacher umfaßt, wobei die Matrix in fester, halbfester bis gelförmiger Konsistenz vorliegt, erhältlich nach dem Verfahren nach Anspruch 1.

11. Pellets nach Anspruch 10, dadurch gekennzeichnet, daß der Weichmacher ausgewählt ist aus der Gruppe bestehend aus:
Glycerol, Propylenglykol, Polyethylenglykole, Triacetin, Sorbitol, Sorbitangemische, Sorbitollösungen, Glucosesirup, Polyole, Zuckeralkohole; sowie deren Mischungen, wobei der Weichmacher in einer Konzentration von etwa 1-50 Gew.-% (bezogen auf die zu verarbeitende Masse) vorliegt.

12. Pellets nach Anspruch 10, dadurch gekennzeichnet, daß die Matrix ferner einen Resorptionsbeschleuniger (Enhancer), ausgewählt aus der Gruppe bestehend aus: Polyethylenglykol, Propylenglykol, Mono- und Diglyceride, Polyalkohole, Lecithin, Bacitracin, Polyoxyethylen-9-laurylether, Natriumlaurylsulfat, Natriumtaurocholat, Natriumglykocholat, Natriumcholat, Natriumtaurodesoxycholat, Natriumglykodesoxycholat, Natriumdesoxycholat, Natriumtaurodihydrofusidat, Ethylendiamintetraessigsäure -Dinatrium, Citronensäure, Dimethyl-beta-Cyclodextrin, Ölsäure, Protease-Inhibitoren, Polyacrylsäure, sowie deren Mischungen, enthält.

13. Pellets nach Anspruch 10, dadurch gekennzeichnet, daß der Gehalt der Matrix an Resorptionsbeschleuniger weniger als 25 Gew.-% (bezogen auf die zu verarbeitende Masse) beträgt.

14. Pellets nach Anspruch 10, dadurch gekennzeichnet, daß die Matrix einen pharmazeutisch akzeptablen Hilfs- oder Trägerstoff, ausgewählt aus der Gruppe bestehend aus: Albumin, Agar-Agar, Gummi-arabicum, Pektine, Traganth, Xanthan, natürliche sowie modifizierte Stärken, Dextrane, Dextrine, Maltodextrin, Chitosan, Alginate, Cellulosederivate, Dextran, Zucker, Glycin, Lactose, Mannit, Polyvinylpyrrolidon, Polyacrylsäure, Polymere aus Methacrylsäure, Polymere aus Methacrylsäureestern, azo-vernetzte Polymethacrylate, Polyurethan-Zucker-Copolymere, wobei als Zuckerkomponente insbesondere oligomere Galactomannane oder Galactomannanderivate geeignet sind, die dann mit aliphatischen Diisocyanaten vernetzt werden, Galactomannanderivate wie Ethyl- oder Acetylgalactomannane, mit Adipinsäure vernetzte Polysaccharide und deren Mischungen enthält.

15. Pellets nach Anspruch 10, gekennzeichnet durch eine Korngröße von 0,2 - 12 mm.

16. Pellets nach Anspruch 10, dadurch gekennzeichnet, daß der Gerüstbildner ein Sol-Gel-Bildner ist, ausgewählt aus der Gruppe bestehend aus:
Gelatine, Gelatine mit einem Maximum der Molekulargewichtsverteilung oberhalb 10⁵ D.

17. Verwendung der Pellets nach Anspruch 10 zur Herstellung einer bioadhäsiven pharmazeutischen Zubereitung.

18. Verwendung der Pellets nach Anspruch 10 zur Herstellung einer peroralen Retardzubereitung.

19. Verwendung der Pellets nach Anspruch 10 in der Pharmazie.

20. Perorale Applikationsform für Peptidarzneistoffe enthaltende Formkörper, gekennzeichnet durch eine Dispersion wenigstens eines Peptidarzneistoffs in einer Matrix, die im wesentlichen Gerüstbildner aus hydrophilen Makromolekülen umfaßt, welche ausgewählt sind aus der Gruppe bestehend aus:
Kollagen, Gelatine, fraktionierte Gelatine, Gelatinederivate, Pflanzenproteinen, Pflanzenproteinhydrolysaten, Kollagenderivaten Kollagenhydrolysaten, Elastinhydrolysaten; sowie deren Mischungen, und welche ferner wenigstens einen Weichmacher umfaßt, wobei die Matrix in fester, halbfester bis gelförmiger Konsistenz vorliegt, erhältlich nach dem Verfahren nach Anspruch 1.

21. Verwendung einer Kombination aus wenigstens einem hydrophilen Makromolekül, ausgewählt aus der Gruppe bestehend aus:
Kollagen, Gelatine, fraktionierte Gelatine, Kollagenderivate, Kollagenhydrolysate, Pflanzenproteine, Pflanzenproteinhydrolysate, Elastinhydrolysate, sowie deren Mischungen und einem Weichmacher, der ausgewählt ist aus der Gruppe bestehend aus:
Glycerol, Propylenglykol, Polyethylenglykole, Triacetin, Sorbitol, Sorbitangemische, Sorbitollösungen, Glucosesirup, Polyole, Zuckeralkohole, sowie deren Mischungen, als Enhancer in einer peroralen Applikationsform nach einem der Ansprüche 10 bis 16 und 20.

## Claims

1. Process for the preparation of an oral administration form in the form of pellets which contain at least one peptide pharmaceutical, characterized in that
a) the peptide pharmaceutical is dispersed in a matrix of a structure-forming agent of hydrophilic macromolecules, which is selected from the group consisting of:
collagen, gelatine, fractionated gelatine, gelatine derivatives, vegetable proteins, vegetable protein hydrolysates, collagen derivatives, collagen hydrolysates, elastin hydrolysates, and mixtures thereof, and plasticizer, and
b) the resulting mixture of structure-forming agents, plasticizer and peptide pharmaceutical is added dropwise to an extremely cold inert liquefied gas and pellets are thus formed.

2. Process according to Claim 1, characterized in that the dispersion is added dropwise to liquid nitrogen.

3. Process according to Claim 1 or 2, characterized in that drops in a uniform predetermined shape are prepared from the dispersion by means of a metering system.

4. Process according to one of Claims 1 - 3, characterized in that plasticizers selected from the group consisting of: glycerol, propylene glycol, polyethylene glycols, triacetin, sorbitol, sorbitan mixtures, sorbitol solutions, glucose syrup and other polyols or sugar alcohols, and mixtures thereof, are added to the dispersion of structure-forming agent and peptide pharmaceutical in a concentration of 1 - 50% by weight (relative to the mass to be administered).

5. Process according to one of Claims 1 - 4, characterized in that the dispersion of structure-forming agent, plasticizer and peptide pharmaceutical is treated with an absorption accelerator selected from the group consisting of: polyethylene glycol, propylene glycol, mono- and diglycerides, polyalcohols, lecithin, bacitracin, polyoxyethylene-9-lauryl ether, sodium lauryl sulphate, sodium taurocholate, sodium glycocholate, sodium cholate, sodium taurodeoxycholate, sodium glycodeoxycholate, sodium deoxycholate, sodium taurodihydrofusidate, ethylenediaminetetraacetic acid-disodium, citric acid, dimethyl-beta-cyclodextrin, oleic acid, protease inhibitors, polyacrylic acid; and mixtures thereof.

6. Process according to one of Claims 1 - 5, characterized in that the matrix is treated with an additional auxiliary or excipient selected from the group consisting of: collagen hydrolysates, elastin, elastin hydrolysates, vegetable proteins, vegetable protein hydrolysates; and mixtures thereof.

7. Process according to Claim 1, characterized in that the pellets according to stage b) are dried.

8. Process according to Claim 1, characterized in that the pellets according to stage b) are freeze-dried.

9. Process according to Claim 1, characterized in that the structure-forming agent employed is gelatine having a maximum in the molecular weight distribution above 10⁵ D at at most 40°C.

10. Oral administration form for peptide pharmaceuticals in the form of pellets, comprising a dispersion of at least one peptide pharmaceutical in a matrix which essentially comprises structure-forming agents of hydrophilic macromolecules which are selected from the group consisting of: collagen, gelatine, fractionated gelatine, gelatine derivatives, vegetable proteins, vegetable protein hydrolysates, collagen derivatives, collagen hydrolysates, elastin hydrolysates, and mixtures thereof, and which further comprises a plasticizer, the matrix being present in solid, semi-solid to gelatinous consistency, obtainable by the process according to Claim 1.

11. Pellets according to Claim 10, characterized in that the plasticizer is selected from the group consisting of:
glycerol, propylene glycol, polyethylene glycols, triacetin, sorbitol, sorbitan mixtures, sorbitol solutions, glucose syrup, polyols, sugar alcohols; and mixtures thereof, the plasticizer being present in a concentration of approximately 1 - 50% by weight (based on the mass to be administered).

12. Pellets according to Claim 10, characterized in that the matrix further contains an absorption accelerator (enhancer) selected from the group consisting of: polyethylene glycol, propylene glycol, mono- and diglycerides, polyalcohols, lecithin, bacitracin, polyoxyethylene-9-lauryl ether, sodium lauryl sulphate, sodium taurocholate, sodium glycocholate, sodium cholate, sodium taurodeoxycholate, sodium glycodeoxycholate, sodium deoxycholate, sodium taurodihydrofusidate, ethylenediaminetetraacetic acid-disodium, citric acid, dimethyl-beta-cyclodextrin, oleic acid, protease inhibitors, polyacrylic acid, and mixtures thereof.

13. Pellets according to Claim 10, characterized in that the content of the matrix in the absorption accelerator is less than 25% by weight (based on the mass to be processed).

14. Pellets according to Claim 10, characterized in that the matrix contains a pharmaceutically acceptable auxiliary or excipient selected from the group consisting of:
albumin, agar-agar, gum arabic, pectins, tragacanth, xanthan, natural and modified starches, dextrans, dextrins, maltodextrin, chitosan, alginates, cellulose derivatives, dextran, sugar, glycine, lactose, mannitol, polyvinylpyrrolidone, polyacrylic acid, polymers of methacrylic acid, polymers of methacrylic acid esters, azo-linked polymethacrylates, polyurethane-sugar copolymers, suitable sugar components in particular being oligomeric galactomannans or galactomannan derivatives which are then linked to aliphatic diisocyanates; galactomannan derivatives such as ethyl- or acetylgalactomannans, polysaccharides linked to adipic acid and mixtures thereof.

15. Pellets according to Claim 10, characterized by a particle size of 0.2 - 12 mm.

16. Pellets according to Claim 10, characterized in that the structure-forming agent is a sol/gel-forming agent selected from the group consisting of:
gelatine, gelatine having a maximum in the molecular weight distribution above 10⁵ D.

17. Use of the pellets according to Claim 10 for the production of a bioadhesive pharmaceutical preparation.

18. Use of the pellets according to Claim 10 for the production of an oral sustained-release preparation.

19. Use of the pellets according to Claim 10 in pharmacy.

20. Oral administration form for moulded articles comprising peptide pharmaceuticals, characterized by a dispersion of at least one peptide pharmaceutical in a matrix which essentially comprises structure-forming agents of hydrophilic macromolecules which are selected from the group consisting of:
collagen, gelatine, fractionated gelatine, gelatine derivatives, vegetable proteins, vegetable protein hydrolysates, collagen derivatives, collagen hydrolysates, elastin hydrolysates; and mixtures thereof, and which further comprises at least one plasticizer, the matrix being present in solid, semi-solid to gelatinous consistency, obtainable by the process according to Claim 1.

21. Use of a combination of at least one hydrophilic macromolecule, selected from the group consisting of: collagen, gelatine, fractionated gelatine, collagen derivatives, collagen hydrolysates, vegetable proteins, vegetable protein hydrolysates, elastin hydrolysates, and mixtures thereof, and a plasticizer which is selected from the group consisting of:
glycerol, propylene glycol, polyethylene glycol, triacetin, sorbitol, sorbitan mixtures, sorbitol solutions, glucose syrup, polyols, sugar alcohols, and mixtures thereof, as enhancer in an oral administration form according to one of Claims 10 to 16 and 20.

## Revendications

1. Procédé de production d'une forme d'administration perorale en pastilles qui contiennent au moins un médicament peptidique, caractérisé en ce que
a) on disperse le médicament peptidique dans une matrice formée d'une matière de structuration constituée de macromolécules hydrophiles, que l'on choisit dans le groupe consistant en :
collagène, gélatine, gélatine fractionnée, dérivés de gélatine, protéines végétales, hydrolysats de protéines végétales, dérivés de collagène, hydrolysats de collagène, hydrolysats d'élastine, ainsi que leurs mélanges, et un plastifiant, et
b) on verse en gouttes le mélange obtenu de matière de structuration, de plastifiant et de médicament peptidique dans un gaz liquéfié inerte à basse température et on forme ainsi des pastilles.

2. Procédé suivant la revendication 1, caractérisé en ce qu'on fait couler la dispersion en gouttes dans de l'azote liquide.

3. Procédé suivant la revendication 1 ou 2, caractérisé en ce qu'on produit à partir de la dispersion des gouttes de forme régulièrement prédéterminée au moyen d'un système doseur.

4. Procédé suivant l'une des revendications 1 à 3, caractérisé en ce qu'on ajoute à la dispersion formée de la matière de structuration et du médicament peptidique, un plastifiant choisi dans le groupe consistant en : glycérol, propylèneglycol, polyéthylèneglycols, triacétine, sorbitol, mélanges de sorbitanne, solutions de sorbitol, sirop de glucose et autres polyols ou alcools-sucres, ainsi que leurs mélanges,
à une concentration de 1 à 50 % en poids (par rapport à la masse à mettre en oeuvre).

5. Procédé suivant l'une des revendications 1 à 4, caractérisé en ce qu'on ajoute à la dispersion formée de la matière de structuration, du plastifiant et du médicament peptidique, un accélérateur de résorption choisi dans le groupe consistant en : polyéthylèneglycol, propylèneglycol, mono- et diglycérides, polyalcools, lécithine, bacitracine, éther polyoxyéthylène-9-laurylique, laurylsulfate de sodium, taurocholate de sodium, glycocholate de sodium, cholate de sodium, taurodésoxycholate de sodium, glycodésoxycholate de sodium, désoxycholate de sodium, taurodihydrofusidate de sodium, sel disodique de l'acide éthylènediamine-tétraacétique, acide citrique, diméthyl-bêta-cyclodextrine, acide oléïque, inhibiteurs de protéase, acide polyacrylique ; ainsi que leurs mélanges.

6. Procédé suivant l'une des revendications 1 à 5, caractérisé en ce qu'on ajoute à la matrice une autre substance auxiliaire ou de support choisie dans le groupe consistant en : hydrolysats de collagène, élastine, hydrolysats d'élastine, protéines végétales, hydrolysats de protéines végétales ; ainsi que leurs mélanges.

7. Procédé suivant la revendication 1, caractérisé en ce qu'on sèche les pastilles selon l'étape b).

8. Procédé suivant la revendication 1, caractérisé en ce qu'on lyophylise les pastilles selon l'étape b).

9. Procédé suivant la revendication 1, caractérisé en ce qu'on utilise comme matière de structuration de la gélatine ayant un maximum de répartition de poids moléculaire au-dessus de 10⁵ D à une température maximale de 40°C.

10. Forme d'administration perorale pour médicaments peptidiques en pastilles, contenant une dispersion d'au moins un médicament peptidique dans une matrice qui comprend principalement des matières de structuration formées de macromolécules hydrophiles, qui sont choisies dans le groupe consistant en : collagène, gélatine, gélatine fractionnée, dérivés de gélatine, protéines végétales, hydrolysats de protéines végétales, dérivés de collagène, hydrolysats de collagène, hydrolysats d'élastine, ainsi que leurs mélanges, et qui comprend en outre au moins un plastifiant, la matrice se présentant avec une consistance solide, semi-solide à gélifiée, pouvant être obtenue par le procédé suivant la revendication 1.

11. Pastilles suivant la revendication 10, caractérisées en ce que le plastifiant est choisi dans le groupe consistant en :
glycérol, propylèneglycol, polyéthylèneglycols, triacétine, sorbitol, mélanges de sorbitanne, solutions de sorbitol, sirop de glucose, polyols, alcools-sucres ; ainsi que leurs mélanges, le plastifiant étant présent à une concentration d'environ 1 à 50 % en poids (par rapport à la masse à mettre en oeuvre).

12. Pastilles suivant la revendication 10, caractérisées en ce que la matrice contient en outre un accélérateur de résorption (stimulateur) choisi dans le groupe consistant en : polyéthylèneglycol, propylèneglycol, mono- et diglycérides, polyalcools, lécithine, bacitracine, éther polyoxyéthylène-9-laurylique, laurylsulfate de sodium, taurocholate de sodium, glycocholate de sodium, cholate de sodium, taurodésoxycholate de sodium, glycodésoxycholate de sodium, désoxycholate de sodium, taurodihydrofusidate de sodium, sel disodique d'acide éthylènediamine-tétracétique, acide citrique, diméthyl-bêta-cyclodextrine, acide oléique, inhibiteurs de protéase, acide polyacrylique, ainsi que leurs mélanges.

13. Pastilles suivant la revendication 10, caractérisées en ce que la teneur de la matrice en accélérateur de résorption s'élève à moins de 25 % en poids (par rapport à la masse à mettre en oeuvre).

14. Pastilles suivant la revendication 10, caractérisées en ce que la matrice contient une substance auxiliaire ou de support acceptable du point de vue pharmaceutique, choisie dans le groupe consistant en : albumine, agar-agar, gomme arabique, pectine, gomme adragante, gomme xanthane, amidons naturels ainsi que modifiés, dextranes, dextrines, maltodextrine, chitosane, alginates, dérivés cellulosiques, dextrane, sucres, glycine, lactose, mannitol, polyvinylpyrrolidone, acide polyacrylique, polymères d'acide méthacrylique, polymères d'esters d'acide méthacrylique, polyméthacrylates azo-réticulés, copolymères polyuréthanne-sucre, les composants sucre appropriés étant en particulier des galactomannanes oligomères ou des dérivés de galactomannanes, qui sont ensuite réticulés avec des diisocyanates aliphatiques, des dérivés de galactomannes tels que des éthyl- ou des acétylgalactomannane, des polysaccharides réticulés par l'acide adipique, et leurs mélanges.

15. Pastilles suivant la revendication 10, caractérisées par un diamètre de grain de 0,2 à 12 mm.

16. Pastilles suivant la revendication 10, caractérisées en ce que la matière de structuration est une matière formant un sol-gel, choisie dans le groupe consistant en :
gélatine, gélatine ayant un maximum de répartition de poids moléculaire au-dessus de 10⁵ D.

17. Utilisation des pastilles suivant la revendication 10 pour l'obtention d'une préparation pharmaceutique bioadhésive.

18. Utilisation des pastilles suivant la revendication 10 pour l'obtention d'une préparation perorale à action retardée.

19. Utilisation des pastilles suivant la revendication 10 en pharmacie.

20. Forme d'application perorale pour des corps façonnés contenant des médicaments peptidiques, caractérisée par une dispersion d'au moins un médicament peptidique dans une matrice qui comprend principalement des matières de structuration formées de macromolécules hydrophiles, qui sont choisies dans le groupe consistant en :
collagène, gélatine, gélatine fractionnée, dérivés de gélatine, protéines végétales, hydrolysats de protéines végétales, dérivés de collagène, hydrolysats de collagène, hydrolysats d'élastine ; ainsi que leurs mélanges, qui comprend en outre au moins un plastifiant et dont la matrice se présente avec une consistance solide, semi-solide à gélifiée, obtenue par le procédé suivant la revendication 1.

21. Utilisation d'une association formée d'au moins une macromolécule hydrophile, choisie dans le groupe consistant en :
collagène, gélatine, gélatine fractionnée, dérivés de collagène, hydrolysats de collagène, protéines végétales, hydrolysats de protéines végétales, hydrolysats d'élastine, ainsi que leurs mélanges, et d'un plastifiant qui est choisi dans le groupe consistant en : glycérol, propylèneglycol, polyéthylèneglycols, triacétine, sorbitol, mélanges de sorbitanne, solutions de sorbitol, sirop de glucose, polyols, alcools-sucres, ainsi que leurs mélanges, comme stimulateur dans une forme d'administration perorale selon l'une des revendications 10 à 16 et 20.
